# EUROPEAN PATENT SPECIFICATION

(11) **EP 4 014 895 B1**
(45) Date of publication and mention of the grant of the patent: **05.08.2026**
(21) Application number: 20878806.7
(22) Date of filing: 09.10.2020
(51) Int. Cl.: A61B 17/128

(54) **SURGICAL INSTRUMENT**
CHIRURGISCHES INSTRUMENT
INSTRUMENT CHIRURGICAL

(30) Priority: 23.10.2019 CN 201911012331
(43) Date of publication of application: 22.06.2022
(73) Proprietor: Intocare Medical Technology (Suzhou) Co., Ltd., Suzhou, Jiangsu 215000 (CN)
(72) Inventor: ZHANG, Hui, Suzhou, Jiangsu 215000 (CN); SHEN, Qing, Suzhou, Jiangsu 215000 (CN)
(74) Representative: Gulde & Partner
(86) International application number: PCT/CN2020/119954
(87) International publication number: WO 2021/078014

(56) References cited:
- EP-A1- 3 834 749
- CN-A- 102 247 182
- CN-A- 104 487 005
- CN-A- 105 748 129
- CN-A- 106 073 853
- CN-U- 209 236 257
- US-A1- 2014 005 693
- US-A1- 2018 008 276
- US-A1- 2019 076 148

## Description

The present application claims the priority to Chinese patent application No. 201911012331.6, filed on October 23, 2019.

### TECHNICAL FIELD

At least one embodiment of the present disclosure relates to a surgical instrument.

### BACKGROUND

In order to fully expose the surgical visual field during the surgical operation, it is necessary to ligate the blood vessels around the target tissue to prevent bleeding. The hemostasis technique has become one of the cores of basic operating techniques of the surgical operation, and surgical procedures on any part of the body almost inevitably involve bleeding and hemostasis. Typically, surgical instruments are used to apply ligating clips, and the ligating clips ligate blood vessels surrounding the target tissue. US 2014/005693A1 directs to a surgical device for clipping tissue can include an actuator, such as a handle or a robotic arm, for example, and a replaceable end effector including a plurality of clips contained therein. After the replaceable end effector has been used, the end effector can be detached from the actuator and a new end effector can be operably coupled with the actuator. Each replaceable end effector can include a firing drive for advancing clips into a receiver of the end effector and a crimping drive configured to deform a clip positioned within the receiver. EP3834749A1 directs to a medical instrument, comprising a clamp (3), an operating head (2) and a handle (1). The operating head (2) is connected to the clamp (3) and the handle (1), and the operating head (2) comprises a housing (10), a firing force applying member provided within the housing (10), a firing rod (21) connected to the firing force applying member, an accommodation cavity (22) provided on one side of the firing rod (21), and a cartridge assembly (23) provided within the accommodation cavity (22) for transporting a ligating clip (231) to the clamp (3). The firing rod (21) is connected to the firing force applying member and the clamp (3) respectively, the housing (10) is provided with an inner cavity (11) in communication with the accommodation cavity (22), an opening (12) is provided at one side of the inner cavity (11) on the housing (10), and the cartridge assembly (23) passes through the opening (12) and the inner cavity (11) and is further inserted into the accommodation cavity (22).

US 2018/008276 A1 discloses a reposable surgical clip applier which includes a handle assembly, an endoscopic assembly selectively connectable to a housing of the handle assembly, and a clip cartridge assembly selectively loadable in and connectable to the endoscopic assembly.

### SUMMARY

It is an objective of the present disclosure to provide a surgical instrument.

The objective can be achieved by the independent claim. Further embodiments of the present invention are defined in the corresponding dependent claims..

### BRIEF DESCRIPTION OF THE DRAWINGS

In order to clearly illustrate the technical solution of the embodiments of the present disclosure, the drawings of the embodiments will be briefly described. It is obvious that the described drawings in the following are only related to some embodiments of the present disclosure and thus are not limitative of the present disclosure.
FIG. 1 is a schematic structural view of a surgical instrument according to at least one embodiment of the present disclosure;
FIG. 2 is a schematic structural view of the surgical instrument of FIG. 1 after disassembly;
FIG. 3 is another schematic structural view of the surgical instrument according to at least one embodiment of the disclosure, illustrating a firing drive assembly and a clip delivery drive assembly provided in a housing;
FIG. 4 is yet another schematic structural view of the surgical instrument according to at least one embodiment of the present disclosure, illustrating a firing sleeve and a firing rod which are inserted into the outer sleeve;
FIG. 5 is a partial enlarged view of FIG. 4;
FIG. 6 is a schematic cross-sectional view of FIG. 1;
FIG. 7 is a partial enlarged view of FIG. 6; and
FIG. 8 is another schematic cross-sectional view of FIG. 1, illustrating an inner sleeve inserted into the firing sleeve.

### DETAILED DESCRIPTION

In order to make objects, technical details and advantages of the embodiments of the disclosure apparent, the technical solutions of the embodiments will be described in a clearly and fully understandable way in connection with the drawings related to the embodiments of the disclosure. Apparently, the described embodiments are just a part but not all of the embodiments of the disclosure. Based on the described embodiments herein, those skilled in the art can obtain other embodiment(s), without any inventive work, which should be within the scope of the disclosure.

Unless otherwise defined, all the technical and scientific terms used herein have the same meanings as commonly understood by one of ordinary skill in the art to which the present disclosure belongs. The terms "first," "second," etc., which are used in the description and the claims of the present application for disclosure, are not intended to indicate any sequence, amount or importance, but distinguish various components. The terms "comprise," "comprising," "include," "including," etc., are intended to specify that the elements or the objects stated before these terms encompass the elements or the objects and equivalents thereof listed after these terms, but do not preclude the other elements or objects. "On," "under," "right," "left" and the like are only used to indicate relative position relationship, and when the position of the object which is described is changed, the relative position relationship may be changed accordingly.

For example, during the surgical operation, due to the requirement on a large number of ligating clips, the operator needs to perform clip application operations multiple times, which reduces the work efficiency, increases the operation time, increases the bleeding volume of the patient, and prolongs the recovery time of the patient. Under this circumstance, clip-jaws that can continuously apply ligating clips come into being, but most of the clip-jaws that can continuously deliver ligating clips are disposable and integrated with the working head, which increases medical costs and increases the economic pressure on patients.

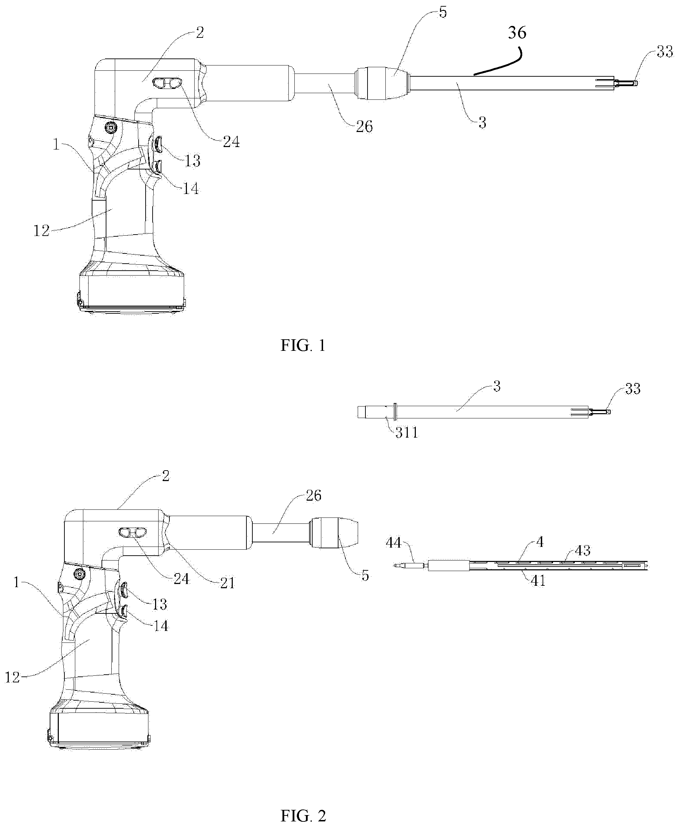

Referring to FIG. 1 to FIG. 3, at least one embodiment of the present disclosure provides a surgical instrument, and the surgical instrument includes a handle 1 and a working head 2 connected with the handle 1, and the handle 1 or the working head 2 provides a power. The surgical instrument further includes a firing sleeve assembly 3 detachably connected with the working head 2, and a clip-jaw 33 provided on a side of the firing sleeve assembly 3 far away from the working head 2. The surgical instrument further includes a clip-cartridge assembly 4 inserted into the firing sleeve assembly 3 and detachably connected with the working head 2. The working head 2 includes: a housing 21; a firing drive assembly 22 provided within the housing 21 for driving the firing sleeve assembly 3 to achieve closing or opening of the clip-jaw 33; a clip delivery drive assembly 23 provided within the housing 21 for driving the clip-cartridge assembly 4 to deliver a clip; and a switching assembly 24 for transmitting the power to the firing drive assembly 22 or the clip delivery drive assembly 23. The firing sleeve assembly 3 is detachably connected (e.g., plugged) with the firing drive assembly 22, and the clip-cartridge assembly 4 is detachably connected (e.g., plugged) with the clip delivery drive assembly 23.

For example, the clip-cartridge assembly 4 is firstly connected with the clip delivery drive assembly 23, and then the firing sleeve assembly 3 is connected with the firing drive assembly 22 so that the firing sleeve assembly 3 encloses the clip-cartridge assembly 4, and the clip-cartridge assembly 4 is docked with the clip-jaw 33. For example, a front end of the clip-cartridge assembly 4 and a rear end of the clip-jaw 33 are in contact with each other. It should be noted that, in the embodiments according to the present disclosure, a front end of a component is an end portion of the component far away from the working head 2, and a rear end of the component is an end portion of the component close to the working head 2.

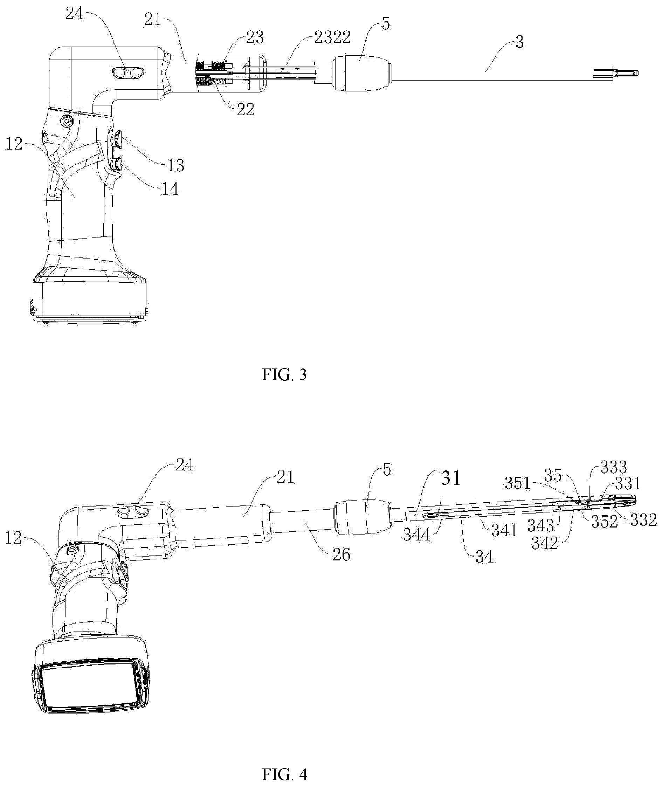

According to embodiments of the invention, referring to FIG. 4 to FIG. 8, the firing sleeve assembly 3 includes a firing sleeve 31 and a firing rod 34. The firing rod 34 includes a first end and a second end, the first end is closer to the working head 2 than the second end, the first end of the firing rod 34 is connected with the firing sleeve 31, and the second end of the firing rod 34 is connected with the clip-jaw 33. The firing sleeve 31 is detachably connected with the firing drive assembly 22, and the firing drive assembly 22 is driven by the power to drive the firing sleeve 31 and the firing rod 34 to move so as to close or open the clip-jaw 33. The firing sleeve assembly 3 further includes an inner sleeve 32 inserted into the firing sleeve 31, the clip-jaw 33 is connected with an end portion of the inner sleeve 32 far away from the working head 2, and the clip-cartridge assembly 4 is inserted into the inner sleeve 32. The firing drive assembly 22 drives the firing sleeve 31 to move, and then the firing sleeve 31 drives the firing rod 34 to move, thereby realizing the closing or opening of the clip-jaw 33 connected with the firing rod 34. During the movement of the firing sleeve 31 and the firing rod 34, the position of the inner sleeve 32 relative to the working head 2 remains unchanged, so as to ensure that the position of the clip-jaw 33 connected with the inner sleeve 32 is stable, which ensures that a ligating clip 42 is applied to the desired position. For example, there are many ways to realize that the position of the inner sleeve 32 relative to the working head 2 remains unchanged, which can be designed according to the actual situation, which is not limited here. For example, the inner sleeve 32 is connected with an outer sleeve 36 as described below, and the outer sleeve 36 is connected with the housing 21 of the working head 2, so that the position of the inner sleeve 32 relative to the working head 2 remains unchanged.

For example, referring to FIG. 1 to FIG. 4, the firing sleeve assembly 3 further includes an outer sleeve 36, the firing sleeve 31 and the firing rod 34 are inserted into the outer sleeve 36, and the outer sleeve 36 is detachably connected with the housing 21 of the working head 2. By providing the outer sleeve 36, the appearance of the surgical instrument is more beautiful. In addition, by rotating the outer sleeve 36, an angle for applying the ligating clip is adjusted, as described below. During the process that the firing drive assembly 22 drives the firing sleeve 31 and the firing rod 34 to move, the position of the outer sleeve 36 relative to the working head 2 remains unchanged. After the assembly of the surgical instrument is completed, the outer sleeve 36 is connected with the housing 21 of the working head 2, so that the position of the outer sleeve 36 relative to the working head 2 remains unchanged. In the firing sleeve assembly 3, the inner sleeve 32, the firing sleeve 31 and the outer sleeve 36 are sequentially arranged from the inside to the outside. For example, the inner sleeve 32 is fixedly connected with the outer sleeve 36. In this case, at the position where the inner sleeve 32 and the outer sleeve 36 are connected with each other, the firing sleeve 31 is correspondingly provided with an avoidance notch to prevent the connection between the inner sleeve 32 and the outer sleeve 36 from hindering the forward and backward movement of the firing sleeve 31 in the axial direction.

For example, the working head 2 further includes a connection tube 26 connected with the housing 21, and at least part of the firing drive assembly 22 and the clip delivery drive assembly 23 are provided within the connection tube 26. In this case, the outer sleeve 36 of the firing sleeve assembly 3 is detachably connected with the connection tube 26. After the clip-cartridge assembly 4 is connected with the clip delivery drive assembly 23, the outer sleeve 36 of the firing sleeve assembly 3 is connected with the connection tube 26, the firing sleeve 31 of the firing sleeve assembly 3 is connected with the firing drive assembly 22, the inner sleeve 32 of the firing sleeve assembly 3 encloses the clip-cartridge assembly 4, and the clip-cartridge assembly 4 is docked with the clip-jaw 33. For example, the connection tube 26 is detachably connected with the housing 21.

For example, referring to FIG. 1, FIG. 2 and FIG. 8, the working head 2 further includes a rotatable head 5 for connecting the connection tube 26 with the outer sleeve 36 of the firing sleeve assembly 3. For example, the rotatable head 5 is rotatably connected with the connection tube 26. For example, the outer sleeve 36 is snap-fitted with the rotatable head 5. For example, one of the rotatable head 5 and the outer sleeve 36 is provided with a hole 311, and the other of the rotatable head 5 and the outer sleeve 36 is provided with a boss 25 which is snap-fitted with the hole 311 so that the outer sleeve 36 is snap-fitted with the rotatable head 5. For example, the rotatable head 5 is rotatably connected with the connection tube 26, for example, the rotatable head 5 is manually rotated, and the rotatable head 5 drives the firing sleeve assembly 3 and the clip-cartridge assembly enclosed in the firing sleeve assembly 3 to rotate to adjust the angle for applying the ligating clip, which is convenient for the operator to operate. For example, the firing sleeve assembly 3 has a limiting member that limits the firing sleeve 31 in the radial direction but does not hinder the forward and backward movement of the firing sleeve 31 in the axial direction from the working head 2 to the clip-jaw 33, so that the rotation of the rotatable head 5 drives the outer sleeve 36 to rotate, and the outer sleeve 36 drives the firing sleeve 31 and the inner sleeve 32 to rotate, thereby realizing the rotation of the entire firing sleeve assembly 3.The radial direction is perpendicular to the axial direction. For example, the limiting member includes a sliding groove provided on one of the outer sleeve 36 and the firing sleeve 31 and extending in the axial direction, and a protrusion provided on the other of the outer sleeve 36 and the firing sleeve 31, and the protrusion is inserted into the sliding groove and is slidable in the axial direction within the sliding groove. Under the action of the limiting member, during the outer sleeve 36 rotates, the firing sleeve 31 is driven to rotate. In addition, because the protrusion is inserted into the sliding groove and is slidable in the axial direction within the sliding groove, the firing sleeve 32 still freely moves backward and forward in the axial direction even if the limiting member is provided.

For example, the firing rod 34 is provided inside the firing sleeve 31 or outside the firing sleeve 31. As mentioned above, the inner sleeve 32 is inserted into the firing sleeve 31; so for the convenience of manufacture, the firing rod 34 is provided outside the firing sleeve 31.

For example, one of the firing rod 34 and the firing sleeve 31 is provided with a bump 321, and the other of the firing rod 34 and the firing sleeve 31 is provided with a long slot 344 that slidably cooperates with the bump 321, and the bump 321 and the long slot 344 are located between the first end of the firing rod 34 and the second end of the firing rod 34. The long slot 344 is engagement with the bump 321 in a slidable manner to prevent the firing rod 34 from deviating from its movement track during the movement, resulting in unstable closing or opening of the clip-jaw 33.

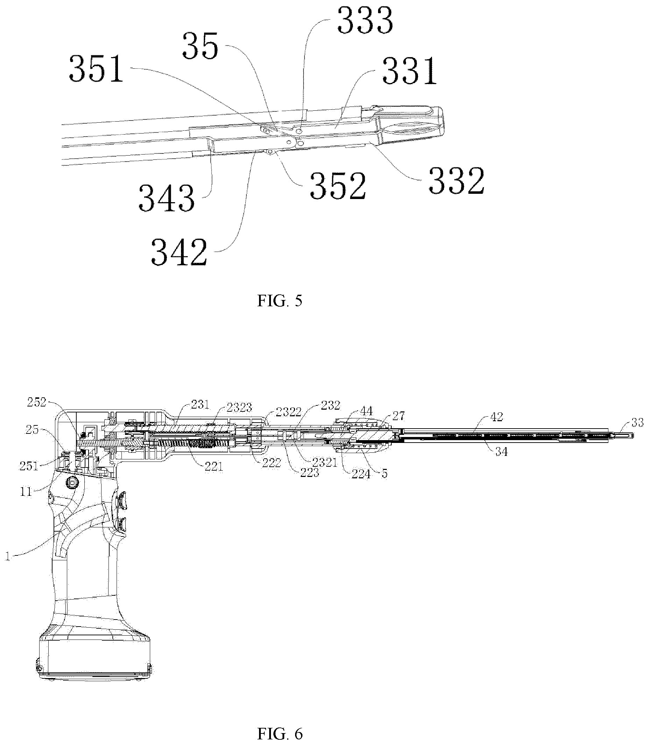

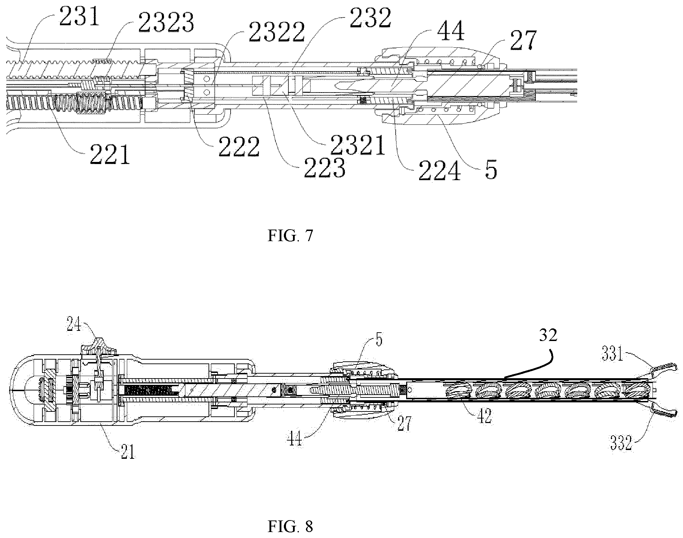

For example, referring to FIG. 5 and FIG. 8, the clip-jaw 33 includes a first clip-jaw portion 331 and a second clip-jaw portion 332 which are connected with the inner sleeve 32, the inner sleeve 32 is provided with a rotating shaft, the first clip-jaw portion 331 and the second clip-jaw portion 332 are respectively provided with a through hole 333, and the first clip-jaw portion 331 and the second clip-jaw portion 332 are opened and closed through the rotation cooperation of the through hole 333 and the rotating shaft. For example, in order to allow the clip-jaw 33 to be better connected with the firing rod 34 and allow the clip-jaw 33 to be fired better, the first clip-jaw portion 331 has a first extension extending from the through hole 333 to the working head 2, and the second clip-jaw portion 332 has a second extension extending from the through hole 333 to the working head 2. The firing rod 34 is connected with the first extension and the second extension through a connecting member 35, the connecting member 35 includes a first connecting member 351 connected with the first extension and a second connecting member 352 connected with the second extension, and the first connecting member 351 and the second connecting member 352 are connected with each other and then are connected with the firing rod 34. The firing rod 34 moves forward to close the first clip-jaw portion 331 and the second clip-jaw portion 332, and the firing rod 34 moves backward to open the first clip-jaw portion 331 and the second clip-jaw portion 332.

It should be noted that, in FIG. 5, in order to simplify the illustration, only the through hole 333 is marked without making the rotating shaft. However, it should be noted that the rotating shaft cooperates with the through hole 333, and the component inserted into the through hole 333 is the rotating shaft.

For example, the firing rod 34 includes a first firing rod portion 341, a second firing rod portion 342 and a bent portion 343 for connecting the first firing rod portion 341 and the second firing rod portion 342. The first firing rod portion 341 is connected with the firing sleeve 31, and the second firing rod portion 342 is connected with the clip-jaw 33, for example, the second firing rod portion 342 is connected with the clip-jaw 33 through the connecting member 35 mentioned above. During the movement of the firing sleeve 31, the firing sleeve 31 drives the first firing rod portion 341, the bent portion 343 and the second firing rod portion 342 to move forward and backward simultaneously. During the forward and backward movement of the firing sleeve 31 in the axial direction, a front end of the inner sleeve 32 may be exposed from a front end of the firing sleeve 31. For example, the bent portion 343 is bent from a side close to the inner sleeve 32 to a side far away from the inner sleeve 32 so that the second firing rod portion 342 does not directly contact the inner sleeve 32, which reduces the friction between the firing rod 34 and the inner sleeve 32 to facilitate firing.

For example, the firing drive assembly 22 includes a firing block 222 connected with the firing sleeve 31 of the firing sleeve assembly 3 and a firing shaft 221 connected with the firing block 222, and the firing shaft 221 is connected with the switching assembly 24. For example, the firing drive assembly 22 includes a driving rod 223 and a firing ring 224, the driving rod 223 is configured for connecting the firing block 222 and the firing ring 224, and the firing sleeve 31 is connected with the firing ring 224. For example, the firing drive assembly 22 further includes other connecting components for connecting the firing block 222 and the firing sleeve 31, and other connecting components for connecting the firing block 222 and the firing shaft 221, which are not specifically limited here, and are determined according to actual conditions.

For example, referring to FIG. 2 and FIG. 8, the clip-cartridge assembly 4 includes a clip tray 41 for placing the ligating clip 42, a clip-pushing piece 43 for pushing the ligating clip 42 to move, and a pushing rod 44 connected with the clip-pushing piece 43. The pushing rod 44 is detachably connected with the clip delivery drive assembly 23. Under the action of the power, the clip delivery drive assembly 23 drives the pushing rod 44 to move, and then drives the clip-pushing piece 43 to move, thereby pushing the ligating clip 42 to move to send the ligating clip 42 into the clip-jaw 33.

For example, during the process that the clip-pushing piece 43 pushes the ligating clip 42 to move, the position of the clip tray 41 relative to the working head 2 remains unchanged. There are many ways to realize that the position of the clip tray 41 relative to the working head 2 remains unchanged, which can be designed according to the actual situation. For example, in the process of connecting the clip-cartridge assembly 4 with the clip delivery drive assembly 23, the inner sleeve 31 is snap-fitted with the clip tray 41 to fix the clip tray 41.

For example, referring to FIG. 6 and FIG. 7, the clip delivery drive assembly 23 includes a plug portion 232 detachably connected with the pushing rod 44 and a clip delivery shaft 231 connected with the plug portion 232, and the clip delivery shaft 231 is connected with the switching assembly 24. Further, for example, the plug portion 232 includes a plug block 2321 detachably connected with the pushing rod 44, a connecting rod 2322 connected with the plug block 2321, and a connecting portion 2323 for connecting the clip delivery shaft 231 and the connecting rod 2322. It should be noted that the plug portion 232 may be other structures connecting the clip delivery shaft 231 and the pushing rod 44, and the specific structure of the plug portion 232 is designed according to the actual situation and is not specifically limited here.

As mentioned above, the handle 1 or the working head 2 provides the power. For example, in the case where the handle 1 provides the power, the power provided by the handle 1 is transmitted to the working head 2, and the working head 2 transmits the power to the firing drive assembly 22 or the clip delivery drive assembly 23. For example, in the case where the working head 2 provides the powered, the handle 1 does not provide the power, and the power is provided by the working head 2 itself and transmitted to the firing drive assembly 22 or the clip delivery drive assembly 23.

For example, the handle 1 is detachably connected with the working head 2. In this case, the handle 1 provides the power so that the handle 1 is capable of being adapted to various different working heads 2.

For example, referring to FIG. 1, FIG. 2 and FIG. 6, a motor 11 is provided in the handle 1, the motor 11 has an output shaft, and the working head 2 further includes a transmission mechanism 25 provided in the housing 21 and configured for connecting the output shaft and the switching assembly 24. The transmission mechanism 25 converts a radial force output by the motor 11 into an axial force, and at the same time transmits the axial force to the switching assembly 24. If the switching assembly 24 transmits the axial force to the clip delivery drive assembly 23, the clip delivery drive assembly 23 drives the clip-cartridge assembly 4 to send the ligating clip to the clip-jaw 33. If the switching assembly 24 transmits the axial force to the firing drive assembly 22, the firing drive assembly 22 drives the firing sleeve 31, which in turn drives the firing rod 34, so that the clip-jaw 33 is closed or opened. For example, in the embodiments of the present disclosure, the transmission mechanism 25 includes a bevel gear 251 connected with the motor 11 and a driving shaft 252 connected with the bevel gear 251.

For example, the handle 1 further includes a handle housing 12 defining a receiving cavity, a control circuit provided in the receiving cavity and electrically connected with the motor 11, and a first switch 13 and a second switch 14 which are provided on the handle housing 12 and coupled to the control circuit. The switching assembly 24 is coupled to the control circuit. For example, the switch assembly 24 is toggled to allow the surgical instrument according to the embodiments of the present disclosure to be in a clip delivery state. The first switch 13 is pressed, the motor 11 provides the power and transmits the power to the clip delivery drive assembly 23, and the clip delivery drive assembly 23 drives the pushing rod 44 of the clip-cartridge assembly 4 to move forward to send the ligating clip 42 into the clip-jaw 33, so as to complete the delivery of the ligating clip. The first switch 13 is released, the pushing rod 44 of the clip-cartridge assembly 4 moves backward to reset. For example, after the delivery of the ligating clip is completed, the switching assembly 24 is toggled to allow the surgical instrument according to the embodiments of the present disclosure in a firing state, the second switch 14 is pressed, and the motor 11 provides the power and transmits the power to drive the firing drive assembly 22. The firing drive assembly 22 drives the firing sleeve 31 and the firing rod 34 to move forward to close the clip-jaw 33, thereby applying the ligating clip 42 to the blood vessel around the target tissue to stop bleeding. The second switch 14 is released, the firing sleeve 31 and the firing rod 34 move backward to reset. For example, a plurality of ligating clips 42 are applied to the blood vessel around the target tissue by repeatedly performing the above clip delivery action and the above firing action. After all of the ligating clips 42 in the clip-cartridge assembly 44 are applied, the surgical instrument according to the embodiments of the present disclosure is disassembled to replace the clip-cartridge assembly 4 with a new one. For example, the control circuit, the first switch 13 and the second switch 14 may be provided in the working head, which is determined according to the actual situation, and is not specifically limited here.

According to the embodiments of the present disclosure, by detachably connecting the firing sleeve assembly 3 and the clip-cartridge assembly 4 with the working head 2, respectively, the clip-cartridge assembly 4 is replaced independently while the firing sleeve assembly 3 and the working head 2 are reused, thereby saving medical costs and reducing the financial pressure on patients. In addition, according to the embodiments of the present disclosure, by providing the switching assembly 24, the power is transmitted to the firing drive assembly 22 connected with the firing sleeve assembly 3 or the clip delivery drive assembly 23 connected with the clip-cartridge assembly 4, so as to control the delivery of ligating clip and the firing of the ligating clip, respectively, thereby preventing the operation errors in the surgical operation to avoid medical accidents.

The foregoing descriptions are merely exemplary implementations of the present disclosure, and are not used to limit the protection scope of the present disclosure, which is determined by the appended claims.

## Claims

1. A surgical instrument, comprising:
a handle (1) and a working head (2) connected with the handle (1), wherein the handle (1) or the working head (2) provides a power;
a firing sleeve assembly (3) detachably connected with the working head (2), wherein a clip-jaw (33) is provided on a side of the firing sleeve assembly (3) far away from the working head (2); and
a clip-cartridge assembly (4) inserted into the firing sleeve assembly (3) and detachably connected with the working head (2), wherein
the working head (2) comprises a housing (21), a firing drive assembly (22) provided within the housing (21) for driving the firing sleeve assembly (3) to achieve closing or opening of the clip-jaw (33), a clip delivery drive assembly (23) provided within the housing (21) for driving the clip-cartridge assembly (4) to deliver a ligating clip (42), and a switching assembly (24) for transmitting the power to the firing drive assembly (22) or the clip delivery drive assembly (23); and
the firing sleeve assembly (3) is detachably connected with the firing drive assembly (22), and the clip-cartridge assembly (4) is detachably connected with the clip delivery drive assembly (23), **characterized in that**
the firing sleeve assembly (3) comprises a firing sleeve (31), an inner sleeve (32), a firing rod (34), and an outer sleeve (36), the firing sleeve (31) and the firing rod (34) are inserted into the outer sleeve (36), and the outer sleeve (36) is detachably connected with the housing (21) of the working head (2), and
the firing rod (34) comprises a first end and a second end, the first end is closer to the working head (2) than the second end, the first end of the firing rod (34) is connected with the firing sleeve (31), the second end of the firing rod (34) is connected with the clip-jaw (33), the firing sleeve (31) is detachably connected with the firing drive assembly (22), and under action of the power, the firing drive assembly (22) drives the firing sleeve (31) and the firing rod (34) to move so as to close or open the clip-jaw (33);
the inner sleeve (32) is inserted into the firing sleeve (31), the clip-jaw (33) is connected with an end portion of the inner sleeve (32) far away from the working head (2), the clip-cartridge assembly (4) is inserted into the inner sleeve (32); and
during a process that the firing drive assembly (22) drives the firing sleeve (31) and the firing rod (34) to move, a position of the inner sleeve (32) relative to the working head (2) remains unchanged; and
during the process that the firing drive assembly (22) drives the firing sleeve (31) and the firing rod (34) to move, a position of the outer sleeve (36) relative to the working head (2) remains unchanged.

2. The surgical instrument according to claim 1, wherein
the clip-cartridge assembly (4) comprises a clip tray (41) for placing the ligating clip (42), a clip-pushing piece (43) for pushing the ligating clip (42) to move, and a pushing rod (44) connected with the clip-pushing piece (43), and the pushing rod (44) is detachably connected with the clip delivery drive assembly (23);
under action of the power, the clip delivery drive assembly (23) drives the pushing rod (44) and the clip-pushing piece (43) to move to push the ligating clip (42) to move; and
during a process that the clip-pushing piece (43) pushes the ligating clip (42) to move, a position of the clip tray (41) relative to the working head (2) remains unchanged.

3. The surgical instrument according to claim 2, wherein the clip delivery drive assembly (23) comprises a plug portion detachably connected with the pushing rod (44) and a clip delivery shaft connected with the plug portion, and the clip delivery shaft is connected with the switching assembly (24).

4. The surgical instrument according to claim 1, wherein the inner sleeve (32) is fixedly connected with the outer sleeve (36), and wherein at a position where the inner sleeve (32) and the outer sleeve (36) are connected with each other the firing sleeve (31) is correspondingly provided with an avoidance notch to prevent the connection between the inner sleeve (32) and the outer sleeve (36) from hindering the forward and backward movement of the firing sleeve (31) in an axial direction.

5. The surgical instrument according to claim 1, wherein the outer sleeve (36) is fixedly connected with the inner sleeve (32).

6. The surgical instrument according to claim 1, wherein the working head (2) further comprises a connection tube (26) connected with the housing (21), at least part of the firing drive assembly (22) and the clip delivery drive assembly (23) are further provided within the connection tube (26), and the outer sleeve (36) is detachably connected with the connection tube (26).

7. The surgical instrument according to claim 6, wherein the connection tube (26) is detachably connected with the housing (21).

8. The surgical instrument according to claim 6, wherein the working head (2) further comprises a rotatable head (5), the rotatable head (5) is rotatably connected with the connection tube (26), and the outer sleeve (36) is snap-fitted with the rotatable head (5).

9. The surgical instrument according to claim 8, wherein one of the rotatable head (5) and the outer sleeve (36) is provided with a hole (311), and the other of the rotatable head (5) and the outer sleeve (36) is provided with a boss (25) which is snap-fitted with the hole (311) so that the outer sleeve (36) is snap-fitted with the rotatable head (5).

10. The surgical instrument according to any one of claims 1 and 5-9, wherein the firing rod (34) is provided within the firing sleeve (31) or outside the firing sleeve (31).

11. The surgical instrument according to any one of claims 1 and 5-10, wherein
the firing rod (34) comprises a first firing rod portion, a second firing rod portion and a bent portion for connecting the first firing rod portion and the second firing rod portion, the first firing rod portion is connected with the firing sleeve (31), and the second firing rod portion is connected with the clip-jaw (33); and
the bent portion is bent from a side close to the inner sleeve (32) to a side away from the inner sleeve (32) so that the second firing rod portion does not directly contact the inner sleeve (32).

12. The surgical instrument according to any one of claims 1 and 5-11, wherein one of the firing rod (34) and the firing sleeve (31) is provided with a bump (321), the other of the firing rod (34) and the firing sleeve (31) is provided with a long slot (344) that slidably cooperates with the bump (321), and the bump (321) and the long slot (34) are located between the first end of the firing rod (34) and the second end of the firing rod (34).

13. The surgical instrument according to any one of claims 1 and 5-12, wherein the firing drive assembly (22) comprises a firing block (222) connected with the firing sleeve (31) and a firing shaft (221) connected with the firing block (222), and the firing shaft (221) is connected with the switching assembly (24).

14. The surgical instrument according to any one of claims 1-3 and 5-13, wherein the handle (1) provides the power, a motor is provided in the handle (1), the motor has an output shaft, and the working head (2) further comprises a transmission mechanism provided in the housing (21) and configured for connecting the output shaft and the switching assembly (24).

15. The surgical instrument according to claim 1, wherein the clip-jaw (33) comprises a first clip-jaw portion (331) and a second clip-jaw portion (332), the inner sleeve (32) comprises a rotating shaft, the first clip-jaw portion (331) and the second clip-jaw portion (332) are respectively provided with a through hole (333), and the first clip-jaw portion (331) and the second clip-jaw portion (332) are opened and closed through the rotation cooperation of the through hole (333) and the rotating shaft of the inner sleeve (32).

## Patentansprüche

1. Chirurgisches Instrument, umfassend:
einen Handgriff (1) und einen mit dem Handgriff (1) verbundenen Arbeitskopf (2), wobei der Handgriff (1) oder der Arbeitskopf (2) eine Leistung bereitstellt;
eine lösbar mit dem Arbeitskopf (2) verbundene Auslösehülsenbaugruppe (3), wobei eine Clipbacke (33) an einer vom Arbeitskopf (2) abgewandten Seite der Auslösehülsenbaugruppe (3) angeordnet ist; und
eine in die Auslösehülsenbaugruppe (3) eingesetzte und lösbar mit dem Arbeitskopf (2) verbundene Clipmagazinbaugruppe (4), wobei
der Arbeitskopf (2) ein Gehäuse (21), eine innerhalb des Gehäuses (21) angeordnete Auslöseantriebsbaugruppe (22) zum Antreiben der Auslösehülsenbaugruppe (3), um ein Schließen oder Öffnen der Clipbacke (33) zu erreichen, eine innerhalb des Gehäuses (21) angeordnete Clipzuführungsantriebsbaugruppe (23) zum Antreiben der Clipmagazinbaugruppe (4), um einen Ligaturclip (42) zuzuführen, und eine Umschaltbaugruppe (24) zum Übertragen der Leistung auf die Auslöseantriebsbaugruppe (22) oder die Clipzuführungsantriebsbaugruppe (23) umfasst; und
wobei die Auslösehülsenbaugruppe (3) lösbar mit der Auslöseantriebsbaugruppe (22) verbunden ist und die Clipmagazinbaugruppe (4) lösbar mit der Clipzuführungsantriebsbaugruppe (23) verbunden ist, **dadurch gekennzeichnet, dass**
die Auslösehülsenbaugruppe (3) eine Auslösehülse (31), eine Innenhülse (32), eine Auslösestange (34) und eine Außenhülse (36) umfasst, die Auslösehülse (31) und die Auslösestange (34) in die Außenhülse (36) eingesetzt sind, und die Außenhülse (36) lösbar mit dem Gehäuse (21) des Arbeitskopfs (2) verbunden ist, und
die Auslösestange (34) ein erstes Ende und ein zweites Ende umfasst, das erste Ende sich näher am Arbeitskopf (2) befindet als das zweite Ende, das erste Ende der Auslösestange (34) mit der Auslösehülse (31) verbunden ist, das zweite Ende der Auslösestange (34) mit der Clipbacke (33) verbunden ist, die Auslösehülse (31) lösbar mit der Auslöseantriebsbaugruppe (22) verbunden ist, und unter Wirkung der Leistung die Auslöseantriebsbaugruppe (22) die Auslösehülse (31) und die Auslösestange (34) zur Bewegung antreibt, um die Clipbacke (33) zu schließen oder zu öffnen;
und dass die Innenhülse (32) in die Auslösehülse (31) eingesetzt ist, die Clipbacke (33) mit einem vom Arbeitskopf (2) abgewandten Endabschnitt der Innenhülse (32) verbunden ist, die Clipmagazinbaugruppe (4) in die Innenhülse (32) eingesetzt ist; und
und wobei während eines Vorgangs, bei dem die Auslöseantriebsbaugruppe (22) die Auslösehülse (31) und die Auslösestange (34) zur Bewegung antreibt, eine Position der Innenhülse (32) bezogen auf den Arbeitskopf (2) unverändert bleibt; und
und wobei während des Vorgangs, bei dem die Auslöseantriebsbaugruppe (22) die Auslösehülse (31) und die Auslösestange (34) zur Bewegung antreibt, eine Position der Außenhülse (36) bezogen auf den Arbeitskopf (2) unverändert bleibt.

2. Chirurgisches Instrument nach Anspruch 1, wobei
die Clipmagazinbaugruppe (4) einen Clipträger (41) zum Platzieren des Ligaturclips (42), ein Clipschiebestück (43) zum Schieben des Ligaturclips (42) zur Bewegung und eine mit dem Clipschiebestück (43) verbundene Schiebestange (44) umfasst, und wobei die Schiebestange (44) lösbar mit der Clipzuführungsantriebsbaugruppe (23) verbunden ist;
wobei unter Wirkung der Leistung die Clipzuführungsantriebsbaugruppe (23) die Schiebestange (44) und das Clipschiebestück (43) zur Bewegung antreibt, um den Ligaturclip (42) zur Bewegung zu schieben; und
und wobei während eines Vorgangs, bei dem das Clipschiebestück (43) den Ligaturclip (42) zur Bewegung schiebt, eine Position des Clipträgers (41) bezogen auf den Arbeitskopf (2) unverändert bleibt.

3. Chirurgisches Instrument nach Anspruch 2, wobei die Clipzuführungsantriebsbaugruppe (23) einen lösbar mit der Schiebestange (44) verbundenen Einsteckabschnitt und eine mit dem Einsteckabschnitt verbundene Clipzuführungswelle umfasst, und wobei die Clipzuführungswelle mit der Umschaltbaugruppe (24) verbunden ist.

4. Chirurgisches Instrument nach Anspruch 1, wobei die Innenhülse (32) fest mit der Außenhülse (36) verbunden ist, und wobei an einer Position, an der die Innenhülse (32) und die Außenhülse (36) miteinander verbunden sind, die Auslösehülse (31) entsprechend mit einer Ausweichkerbe versehen ist, um zu verhindern, dass die Verbindung zwischen der Innenhülse (32) und der Außenhülse (36) die Vorwärts- und Rückwärtsbewegung der Auslösehülse (31) in einer axialen Richtung behindert.

5. Chirurgisches Instrument nach Anspruch 1, wobei die Außenhülse (36) fest mit der Innenhülse (32) verbunden ist.

6. Chirurgisches Instrument nach Anspruch 1, wobei der Arbeitskopf (2) ferner ein mit dem Gehäuse (21) verbundenes Verbindungsrohr (26) umfasst, mindestens ein Teil der Auslöseantriebsbaugruppe (22) und der Clipzuführungsantriebsbaugruppe (23) ferner innerhalb des Verbindungsrohrs (26) angeordnet ist und die Außenhülse (36) lösbar mit dem Verbindungsrohr (26) verbunden ist.

7. Chirurgisches Instrument nach Anspruch 6, wobei das Verbindungsrohr (26) lösbar mit dem Gehäuse (21) verbunden ist.

8. Chirurgisches Instrument nach Anspruch 6, wobei der Arbeitskopf (2) ferner einen drehbaren Kopf (5) umfasst, wobei der drehbare Kopf (5) drehbar mit dem Verbindungsrohr (26) verbunden ist und die Außenhülse (36) mit dem drehbaren Kopf (5) verrastet ist.

9. Chirurgisches Instrument nach Anspruch 8, wobei eines von dem drehbaren Kopf (5) und der Außenhülse (36) mit einem Loch (311) versehen ist und das andere von dem drehbaren Kopf (5) und der Außenhülse (36) mit einem Vorsprung (25) versehen ist, der mit dem Loch (311) verrastet ist, sodass die Außenhülse (36) mit dem drehbaren Kopf (5) verrastet ist.

10. Chirurgisches Instrument nach einem der Ansprüche 1 und 5 bis 9, wobei die Auslösestange (34) innerhalb der Auslösehülse (31) oder außerhalb der Auslösehülse (31) angeordnet ist.

11. Chirurgisches Instrument nach einem der Ansprüche 1 und 5 bis 10, wobei
die Auslösestange (34) einen ersten Auslösestangenabschnitt, einen zweiten Auslösestangenabschnitt und einen gebogenen Abschnitt zum Verbinden des ersten Auslösestangenabschnitts und des zweiten Auslösestangenabschnitts umfasst, der erste Auslösestangenabschnitt mit der Auslösehülse (31) verbunden ist, und der zweite Auslösestangenabschnitt mit der Clipbacke (33) verbunden ist; und
wobei der gebogene Abschnitt von einer der Innenhülse (32) zugewandten Seite zu einer von der Innenhülse (32) abgewandten Seite gebogen ist, sodass der zweite Auslösestangenabschnitt die Innenhülse (32) nicht direkt berührt.

12. Chirurgisches Instrument nach einem der Ansprüche 1 und 5 bis 11, wobei eines von der Auslösestange (34) und der Auslösehülse (31) mit einem Höcker (321) versehen ist, das andere von der Auslösestange (34) und der Auslösehülse (31) mit einem Langloch (344) versehen ist, das gleitend mit dem Höcker (321) zusammenwirkt, und wobei der Höcker (321) und das Langloch (344) sich zwischen dem ersten Ende der Auslösestange (34) und dem zweiten Ende der Auslösestange (34) befinden.

13. Chirurgisches Instrument nach einem der Ansprüche 1 und 5 bis 12, wobei die Auslöseantriebsbaugruppe (22) einen mit der Auslösehülse (31) verbundenen Auslöseblock (222) und eine mit dem Auslöseblock (222) verbundene Auslösewelle (221) umfasst, und wobei die Auslösewelle (221) mit der Umschaltbaugruppe (24) verbunden ist.

14. Chirurgisches Instrument nach einem der Ansprüche 1 bis 3 und 5 bis 13, wobei der Handgriff (1) die Leistung bereitstellt, ein Motor in dem Handgriff (1) angeordnet ist, der Motor eine Abtriebswelle aufweist, und der Arbeitskopf (2) ferner einen Übertragungsmechanismus umfasst, der in dem Gehäuse (21) angeordnet und zum Verbinden der Abtriebswelle und der Umschaltbaugruppe (24) eingerichtet ist.

15. Chirurgisches Instrument nach Anspruch 1, wobei die Clipbacke (33) einen ersten Clipbackenabschnitt (331) und einen zweiten Clipbackenabschnitt (332) umfasst, die Innenhülse (32) eine Drehwelle umfasst, der erste Clipbackenabschnitt (331) und der zweite Clipbackenabschnitt (332) jeweils mit einem Durchgangsloch (333) versehen sind, und der erste Clipbackenabschnitt (331) und der zweite Clipbackenabschnitt (332) durch das Rotationszusammenwirken des Durchgangslochs (333) und der Drehwelle der Innenhülse (32) geöffnet und geschlossen werden.

## Revendications

1. Instrument chirurgical, comprenant :
une poignée (1) et une tête de travail (2) reliée à la poignée (1), dans lequel la poignée (1) ou la tête de travail (2) fournit une puissance ;
un ensemble de manchon de déclenchement (3) relié de manière amovible à la tête de travail (2), dans lequel une mâchoire d'agrafe (33) est prévue sur un côté de l'ensemble de manchon de déclenchement (3) éloigné de la tête de travail (2) ; et
un ensemble de cartouche d'agrafes (4) inséré dans l'ensemble de manchon de déclenchement (3) et relié de manière amovible à la tête de travail (2), dans lequel
la tête de travail (2) comprend un boîtier (21), un ensemble d'entraînement de déclenchement (22) prévu à l'intérieur du boîtier (21) pour entraîner l'ensemble de manchon de déclenchement (3) afin d'obtenir la fermeture ou l'ouverture de la mâchoire d'agrafe (33), un ensemble d'entraînement de distribution d'agrafes (23) prévu à l'intérieur du boîtier (21) pour entraîner l'ensemble de cartouche d'agrafes (4) afin de distribuer une agrafe de ligature (42), et un ensemble de commutation (24) destiné à transmettre la puissance à l'ensemble d'entraînement de déclenchement (22) ou à l'ensemble d'entraînement de distribution d'agrafes (23) ; et
l'ensemble de manchon de déclenchement (3) est relié de manière amovible à l'ensemble d'entraînement de déclenchement (22), et l'ensemble de cartouche d'agrafes (4) est relié de manière amovible à l'ensemble d'entraînement de distribution d'agrafes (23), **caractérisé en ce que**
l'ensemble de manchon de déclenchement (3) comprend un manchon de déclenchement (31), un manchon interne (32), une tige de déclenchement (34) et un manchon externe (36), le manchon de déclenchement (31) et la tige de déclenchement (34) étant insérés dans le manchon externe (36), et le manchon externe (36) étant relié de manière amovible au boîtier (21) de la tête de travail (2), et
la tige de déclenchement (34) comprend une première extrémité et une seconde extrémité, la première extrémité étant plus proche de la tête de travail (2) que la seconde extrémité, la première extrémité de la tige de déclenchement (34) étant reliée au manchon de déclenchement (31), la seconde extrémité de la tige de déclenchement (34) étant reliée à la mâchoire d'agrafe (33), le manchon de déclenchement (31) étant relié de manière amovible à l'ensemble d'entraînement de déclenchement (22), et, sous l'action de la puissance, l'ensemble d'entraînement de déclenchement (22) entraîne le manchon de déclenchement (31) et la tige de déclenchement (34) à se déplacer afin de fermer ou d'ouvrir la mâchoire d'agrafe (33) ;
le manchon interne (32) est inséré dans le manchon de déclenchement (31), la mâchoire d'agrafe (33) est reliée à une partie d'extrémité du manchon interne (32) éloignée de la tête de travail (2), l'ensemble de cartouche d'agrafes (4) est inséré dans le manchon interne (32) ; et
pendant un processus au cours duquel l'ensemble d'entraînement de déclenchement (22) entraîne le manchon de déclenchement (31) et la tige de déclenchement (34) à se déplacer, une position du manchon interne (32) par rapport à la tête de travail (2) reste inchangée ; et
pendant le processus au cours duquel l'ensemble d'entraînement de déclenchement (22) entraîne le manchon de déclenchement (31) et la tige de déclenchement (34) à se déplacer, une position du manchon externe (36) par rapport à la tête de travail (2) reste inchangée.

2. Instrument chirurgical selon la revendication 1, dans lequel
l'ensemble de cartouche d'agrafes (4) comprend un plateau d'agrafes (41) destiné à recevoir l'agrafe de ligature (42), une pièce de poussée d'agrafes (43) destinée à pousser l'agrafe de ligature (42) pour la faire se déplacer, et une tige de poussée (44) reliée à la pièce de poussée d'agrafes (43), et la tige de poussée (44) est reliée de manière amovible à l'ensemble d'entraînement de distribution d'agrafes (23) ;
sous l'action de la puissance, l'ensemble d'entraînement de distribution d'agrafes (23) entraîne la tige de poussée (44) et la pièce de poussée d'agrafes (43) à se déplacer afin de pousser l'agrafe de ligature (42) à se déplacer ; et
pendant un processus au cours duquel la pièce de poussée d'agrafes (43) pousse l'agrafe de ligature (42) à se déplacer, une position du plateau d'agrafes (41) par rapport à la tête de travail (2) reste inchangée.

3. Instrument chirurgical selon la revendication 2, dans lequel l'ensemble d'entraînement de distribution d'agrafes (23) comprend une partie de raccordement reliée de manière amovible à la tige de poussée (44) et un arbre de distribution d'agrafes relié à la partie de raccordement, et l'arbre de distribution d'agrafes est relié à l'ensemble de commutation (24).

4. Instrument chirurgical selon la revendication 1, dans lequel le manchon interne (32) est fixé de manière fixe au manchon externe (36), et dans lequel, à une position où le manchon interne (32) et le manchon externe (36) sont reliés l'un à l'autre, le manchon de déclenchement (31) est pourvu de manière correspondante d'une encoche d'évitement afin d'empêcher que la liaison entre le manchon interne (32) et le manchon externe (36) n'entrave le mouvement vers l'avant et vers l'arrière du manchon de déclenchement (31) dans une direction axiale.

5. Instrument chirurgical selon la revendication 1, dans lequel le manchon externe (36) est fixé de manière fixe au manchon interne (32).

6. Instrument chirurgical selon la revendication 1, dans lequel la tête de travail (2) comprend en outre un tube de connexion (26) relié au boîtier (21), au moins une partie de l'ensemble d'entraînement de déclenchement (22) et de l'ensemble d'entraînement de distribution d'agrafes (23) étant en outre prévue à l'intérieur du tube de connexion (26), et le manchon externe (36) étant relié de manière amovible au tube de connexion (26).

7. Instrument chirurgical selon la revendication 6, dans lequel le tube de connexion (26) est relié de manière amovible au boîtier (21).

8. Instrument chirurgical selon la revendication 6, dans lequel la tête de travail (2) comprend en outre une tête rotative (5), la tête rotative (5) étant reliée de manière rotative au tube de connexion (26), et le manchon externe (36) étant encliqueté sur la tête rotative (5).

9. Instrument chirurgical selon la revendication 8, dans lequel l'un de la tête rotative (5) et du manchon externe (36) est pourvu d'un trou (311), et l'autre de la tête rotative (5) et du manchon externe (36) est pourvu d'un bossage (25) qui est encliqueté dans le trou (311) de sorte que le manchon externe (36) soit encliqueté sur la tête rotative (5).

10. Instrument chirurgical selon l'une quelconque des revendications 1 et 5 à 9, dans lequel la tige de déclenchement (34) est prévue à l'intérieur du manchon de déclenchement (31) ou à l'extérieur du manchon de déclenchement (31).

11. Instrument chirurgical selon l'une quelconque des revendications 1 et 5 à 10, dans lequel
la tige de déclenchement (34) comprend une première partie de tige de déclenchement, une seconde partie de tige de déclenchement et une partie coudée destinée à relier la première partie de tige de déclenchement et la seconde partie de tige de déclenchement, la première partie de tige de déclenchement étant reliée au manchon de déclenchement (31), et la seconde partie de tige de déclenchement étant reliée à la mâchoire d'agrafe (33) ; et
la partie coudée est courbée depuis un côté proche du manchon interne (32) vers un côté éloigné du manchon interne (32) de sorte que la seconde partie de tige de déclenchement n'entre pas directement en contact avec le manchon interne (32).

12. Instrument chirurgical selon l'une quelconque des revendications 1 et 5 à 11, dans lequel l'un de la tige de déclenchement (34) et du manchon de déclenchement (31) est pourvu d'une saillie (321), l'autre de la tige de déclenchement (34) et du manchon de déclenchement (31) est pourvu d'une fente allongée (344) coopérant de manière coulissante avec la saillie (321), et la saillie (321) et la fente allongée (344) sont situées entre la première extrémité de la tige de déclenchement (34) et la seconde extrémité de la tige de déclenchement (34).

13. Instrument chirurgical selon l'une quelconque des revendications 1 et 5 à 12, dans lequel l'ensemble d'entraînement de déclenchement (22) comprend un bloc de déclenchement (222) relié au manchon de déclenchement (31) et un arbre de déclenchement (221) relié au bloc de déclenchement (222), et l'arbre de déclenchement (221) est relié à l'ensemble de commutation (24).

14. Instrument chirurgical selon l'une quelconque des revendications 1 à 3 et 5 à 13, dans lequel la poignée (1) fournit la puissance, un moteur est prévu dans la poignée (1), le moteur possède un arbre de sortie, et la tête de travail (2) comprend en outre un mécanisme de transmission prévu dans le boîtier (21) et configuré pour relier l'arbre de sortie et l'ensemble de commutation (24).

15. Instrument chirurgical selon la revendication 1, dans lequel la mâchoire d'agrafe (33) comprend une première partie de mâchoire d'agrafe (331) et une seconde partie de mâchoire d'agrafe (332), le manchon interne (32) comprend un arbre rotatif, la première partie de mâchoire d'agrafe (331) et la seconde partie de mâchoire d'agrafe (332) étant respectivement pourvues d'un trou traversant (333), et la première partie de mâchoire d'agrafe (331) et la seconde partie de mâchoire d'agrafe (332) étant ouvertes et fermées par la coopération en rotation du trou traversant (333) et de l'arbre rotatif du manchon interne (32).
